# EUROPEAN PATENT APPLICATION

(11) **EP 4 506 030 A1**
(43) Date of publication of application: **12.02.2025**
(21) Application number: 23854979.4
(22) Date of filing: 19.05.2023
(51) Int. Cl.: A61N 1/36, A61N 1/04, A61B 5/374, A61B 5/00, G16H 20/30, A61B 5/024, A61B 5/346

(54) **METHOD FOR APPLYING ELECTRICAL STIMULATION TO VAGUS NERVE, AND VAGUS NERVE STIMULATION DEVICE THAT REFLECT USER'S CONDITION AND STIMULATION SENSITIVITY THROUGH BIOLOGICAL SIGNAL**

(30) Priority: 17.08.2022 KR 20220102865; 17.08.2022 KR 20220102866
(71) Applicant: Neurive Co., Ltd., Juchon-myeon Gimhae-si, Gyeongsangnam-do 50969 (KR)
(72) Inventor: SONG, Jae Jun, Seoul 06501 (KR); CHOI, Hyuk, Seoul 06095 (KR); HONG, Ki Hwan, Seoul 03709 (KR); MOON, Byoung Jong, Siheung-si, Gyeonggi-do 14922 (KR)
(74) Representative: Franke, Dirk
(86) International application number: PCT/KR2023/006860
(87) International publication number: WO 2024/039006

(57) **Abstract**

The present invention relates to a method for applying electrical stimulation to the vagus nerve through an electrode coming into contact with the human skin, the method comprising: an electrical stimulation guideline input step of inputting electrical stimulation guidelines including a numerical value for electrical stimulation; an initial stimulation value setting step of, before applying the electrical stimulation, applying an SPI, calculated by applying one or more of demographic and environmental markers, so as to derive an initial stimulation value obtained by adjusting the numerical value included in the electrical stimulation guidelines; an electrical stimulation starting step of applying the electrical stimulation with the initial stimulation value; and an intra-stimulation adjustment step of measuring a user's biological signal while the electrical stimulation is applied to the user and adjusting the electrical stimulation applied to the electrode, wherein the SPI is an index that quantifies sensitivity to electrical stimulation, the demographic marker is a marker that statistically reflects the degree of sensitivity to electrical stimulation, and the environmental marker is a marker that reflects environmental factors which affect sensitivity to electrical stimulation. The present invention applies electrical stimulation by reflecting sensitivity to the electrical stimulation, and thus has the effect of preempting problems of a user feeling pain due to the electrical stimulation. In addition, the present invention applies the electrical stimulation by reflecting the user's condition by means of heart rate variability, and thus has the effect of preventing electrical stimulation that has a negative impact on the user from being applied

## Description

### [Technical Field]

The present disclosure relates to a method and apparatus for applying electrical stimulation to a vagus nerve and, in more detail, a method and apparatus for applying electrical stimulation to a vagus nerve through an electrode that is in contact with the skin of a human body.

### [Related Art]

The vagus nerve is one of cranial nerves and corresponds to the tenth cranial nerve. The vagus nerve is a mixed nerve coming out from the brain, distributed through the face, chest, and abdomen, and including parasympathetic nerve fibers, and takes part in controlling of parasympathetic nerves acting on the heart, lungs, alimentary canal, etc. The vagus nerve has the longest and the most complicated structure of the cranial nerves and includes all of the sensory nerve fibers and the motor nerve fibers.

Vagus nerve stimulation devices for the treatment of epilepsy and depression have reportedly received approval from the U.S. Food and Drug Administration (FDA) and these devices target the cervical branches positioned in the neck. However, such electrical stimulation of the cervical branches of a vagus nerve involves a procedure of making a direct incision in the skin, exposing the cervical branches of the vagus nerve, winding a coil, which is an electrolyte, around it, and implanting a microchip. Accordingly, it cannot be used in the realm of self-treatment by the general public.

Meanwhile, eastern medicine applies acupuncture on the ear as a method to treat diseases, the ear is an area where auricular branch of the vagus nerve is distributed, and it can be considered that stimulation of the vagus nerve is utilized for treatment.

Accordingly, devices that electrically stimulate a vagus nerve distributed in the ear have been developed for the purposes of alleviating symptoms, etc., but these devices cannot obtain desired effects due to differences in individual sensitivity to electrical stimulation and differences in response of the human body in used, so the devices are not generally used.

### [Disclosure]

### [Technical Problem]

The present disclosure has been made in an effort to solve the problems of the related art described above and an objective of the present disclosure is to provide a method and apparatus for applying electrical stimulation to a vagus nerve with problems accompanying pain or negative effects by reflecting the sensitivity of a user to electrical stimulation and the state of a human body during application of stimulation.

### [Technical Solution]

In order to achieve the objectives, a method for applying electrical stimulation to a vagus nerve according to the present disclosure is a method for applying electrical stimulation to a vagus nerve through an electrode that is in contact with the skin of a human body and includes: an electrical stimulation guideline input step of inputting an electrical stimulation guideline comprising numerical values for electrical stimulation; an initial stimulation value setting step of deriving initial stimulation values adjusting the numerical values comprised in the electrical stimulation guideline by applying an SPI calculated by applying one or more of demographic markers and environmental markers before electrical stimulation is applied; an electrical stimulation starting step of applying electrical stimulation on the basis of the initial stimulation values; and an intra-stimulation adjustment step of adjusting electrical stimulation that is applied to the electrode by measuring a biological signal of a user with electrical stimulation applied to the user, wherein the SPI is an index quantifying sensitivity to electrical stimulation, the demographic markers are markers statistically reflecting sensitivity to electrical stimulation, and the environmental markers are markers reflecting environmental factors influencing sensitivity to electrical stimulation.

The demographic markers may include one or more pieces of information of age, sex, BMI, presence of medication, and presence of disease.

The environmental markers may include one or more pieces of information of temperature, humidity, illuminance, and a discomfort index.

In the initial stimulation value setting step, the input electrical stimulation guideline may not be changed when the SPI is a predetermined value or less, and the electrical stimulation guideline may be changed in proportion to an excess value of the SPI when the SPI exceeds the predetermined value.

The electrical stimulation guideline comprises information about a current and a duty ratio, and the current of the electrical stimulation guideline may be decreased and the duty ratio of the electrical stimulation guideline may be increased in proportion to the excess value of the SPI in the initial stimulation value setting step.

The biological signal of the user measured in the intra-stimulation adjustment step may be a brain wave or heart rate variability.

When a brain wave is used, the intra-stimulation adjustment step may adjust electrical stimulation that is applied to the electrode by applying the amount of variation of an FSWR calculated by measuring a brain wave of a user with electrical stimulation applied to the user; and the amount of variation of the FSWR is for checking influence on a human body by electrical stimulation applied to a user and the FSWR may be calculated by dividing power of a fast wave band with a relatively high frequency by power of a slow wave band with a relatively low frequency in a measured brain wave.

The fast wave band may be a brain wave exceeding 13Hz and the slow wave band may be a brain wave of 13Hz or less.

The amount of variation of the FSWR may use an FSWR change rate that is a ratio of change from an FSWR₀ measured before or immediately after electrical stimulation is applied to an FSWR_{N} measured after electrical stimulation is applied, and when the FSWR change rate is larger than a predetermined value, electrical stimulation that is applied through the electrode may be adjusted.

When the FSWR change rate is larger than a predetermined value, a current of electrical stimulation that is applied through the electrode may be decreased and a duty ratio may be increased.

When the FSWR change rate increases over 50%, the current of electrical stimulation may be decreased into a range of 10 ~ 30% and the duty ratio may be increased into a range of 10 ~ 45%.

When heart rate variability is used, the intra-stimulation adjustment step may adjust electrical stimulation that is applied to the electrode by applying the amount of variation of an FSBR calculated by measuring heart rate variability of a user with electrical stimulation applied to the user; and the amount of variation of the FSBR is for checking influence on a human body by electrical stimulation applied to a user and the FSBR may be calculated by dividing power of a high frequency band with a relatively high frequency by power of a low frequency band with a relatively low frequency in measured heart rate variability.

The high frequency band may be the range of 0.15Hz or more and 0.4Hz or less and the low frequency band may be the range of 0.04Hz or more and less than 0.15Hz.

The amount of variation of the FSBR may an FSBR change rate that is a ratio of change from an FSBR₀ measured before or immediately after electrical stimulation is applied to an FSBR_{N} measured after electrical stimulation is applied, and when the FSBR change rate is larger than a predetermined value, electrical stimulation that is applied through the electrode may be adjusted.

When the FSBR change rate is larger than a predetermined value, a current of electrical stimulation that is applied through the electrode may be decreased and a duty ratio may be increased.

When the FSBR change rate increases over 50%, the current of electrical stimulation may be decreased into a range of 10 ~ 30% and the duty ratio may be increased into a range of 10 ~ 45%.

A device for applying electrical stimulation to a vagus nerve according to another aspect of the present disclosure includes: an electrode configured to apply electrical stimulation in contact with the skin of a human body; an input unit configured to receive an electrical stimulation guideline comprising numerical information about electrical stimulation; an initial stimulation value setter configured to derive initial stimulation values adjusting the numerical information comprised in the electrical stimulation guideline by applying an SPI calculated by applying one or more of demographic markers and environmental markers before electrical stimulation is applied; and an intra-stimulation adjuster configured to adjust electrical stimulation that is applied to the electrode by measuring a biological signal of a user with electrical stimulation applied to the user, wherein the SPI is an index quantifying sensitivity to electrical stimulation, the demographic markers are markers statistically reflecting sensitivity to electrical stimulation, and the environmental markers are markers reflecting environmental factors influencing sensitivity to electrical stimulation.

The demographic markers may include one or more pieces of information of age, sex, BMI, presence of medication, and presence of disease.

The environmental markers may include one or more pieces of information of temperature, humidity, illuminance, and a discomfort index.

The initial stimulation value setter may not change the input electrical stimulation guideline when the SPI is a predetermined value or less, and may change the electrical stimulation guideline in proportion to an excess value of the SPI when the SPI exceeds the predetermined value.

The electrical stimulation guideline may comprise information about a current and a duty ratio, and the current of the electrical stimulation guideline may be decreased and the duty ratio of the electrical stimulation guideline may be increased in proportion to the excess value of the SPI in the initial stimulation value setting step.

The biological signal of the user that is measured by the intra-stimulation adjuster may be a brain wave or heart rate variability.

When a brain wave is used, the intra-stimulation adjuster may adjust electrical stimulation that is applied to the electrode by applying the amount of variation of an FSWR calculated by measuring a brain wave of a user with electrical stimulation applied to the user, and the amount of variation of the FSWR is for checking influence on a human body by electrical stimulation applied to a user and the FSWR may be calculated by dividing power of a fast wave band with a relatively high frequency by power of a slow wave band with a relatively low frequency in a measured brain wave.

The fast wave band may be a brain wave exceeding 13Hz and the slow wave band may be a brain wave of 13Hz or less.

The amount of variation of the FSWR may use an FSWR change rate that is a ratio of change from an FSWR₀ measured before or immediately after electrical stimulation is applied to an FSWR_{N} measured after electrical stimulation is applied, and when the FSWR change rate is larger than a predetermined value, electrical stimulation that is applied through the electrode may be adjusted.

When the FSWR change rate is larger than a predetermined value, a current of electrical stimulation that is applied through the electrode may be decreased and a duty ratio may be increased.

When the FSWR change rate increases over 50%, the current of electrical stimulation may be decreased into a range of 10 ~ 30% and the duty ratio may be increased into a range of 10 ~ 45%.

When heart rate variability is used, the intra-stimulation adjuster may adjust electrical stimulation that is applied to the electrode by applying the amount of variation of an FSBR calculated by measuring heart rate variability of a user with electrical stimulation applied to the user; and the amount of variation of the FSBR is for checking influence on a human body by electrical stimulation applied to a user and the FSBR may be calculated by dividing power of a high frequency band with a relatively high frequency by power of a low frequency band with a relatively low frequency in measured heart rate variability.

The high frequency band may be the range of 0.15Hz or more and 0.4Hz or less and the low frequency band may be the range of 0.04Hz or more and less than 0.15Hz.

The amount of variation of the FSBR may an FSBR change rate that is a ratio of change from an FSBR₀ measured before or immediately after electrical stimulation is applied to an FSBR_{N} measured after electrical stimulation is applied, and when the FSBR change rate is larger than a predetermined value, electrical stimulation that is applied through the electrode may be adjusted.

When the FSBR change rate is larger than a predetermined value, a current of electrical stimulation that is applied through the electrode may be decreased and a duty ratio may be increased.

When the FSBR change rate increases over 50%, the current of electrical stimulation may be decreased into a range of 10 ~ 30% and the duty ratio may be increased into a range of 10 ~ 45%.

### [Advantageous Effects]

The present disclosure configured as described above has an effect that it is possible to prevent the problem that a user feels pain due to electrical stimulation by applying electrical stimulation by reflecting sensitivity to electrical stimulation.

Further, there is an effect that it is possible to prevent the problem that electrical stimulation having negative influence on a user from being applied by applying electrical stimulation by reflecting the condition of the user through a brain wave.

### [Brief Description of Drawings]

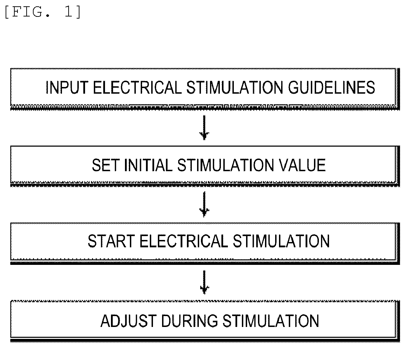

FIG. 1 is a flowchart illustrating a method for applying electrical stimulation to a vagus nerve by reflecting the user's condition and stimulation sensitivity through a biological signal according to the present disclosure.

### [Mode for Invention]

Embodiments of the present disclosure are described in detail with reference to the accompanying drawings.

However, such embodiments of the present disclosure may be modified in various ways and the scope of the present disclosure is not limited only to the embodiments to be described below. The shape, sizes, etc. of elements may be exaggerated in the drawings for clearer description and elements indicated by the same reference numerals in the drawings are the same components.

Further, when an element is referred to as being "connected with" another element throughout the specification, it may be "directly connected" to the other element and may also be "electrically connected" to the other element with another element intervening therebetween. Further, unless explicitly described otherwise, "comprising" or "having" any components will be understood to imply the inclusion of other components rather than the exclusion of any other components.

Terms "first", "second", etc. are provided for discriminating one component from another component and the scope of a right is not limited to the terms. For example, the first component may be named the second component, and vice versa.

FIG. 1 is a flowchart illustrating a method for applying electrical stimulation to a vagus nerve by reflecting the user's condition and stimulation sensitivity through a biological signal according to the present disclosure.

The method for applying electrical stimulation to a vagus nerve of this embodiment performs first an electrical stimulation guideline input step.

An electrical stimulation guideline includes information about electrical stimulation for stimulating a vagus nerve. An electrical stimulation guideline may be determined on the basis diagnosis and prescription by a doctor at hospital or may be derived through a separate program or system. In this case, the electrical stimulation guideline is determined in accordance with the disease or symptoms of a user and may not reflect the characteristics of the user. Accordingly, electrical stimulation included in an electrical stimulation guideline may cause pain to a user, depending on sensitivity to electrical stimulation.

An electrical stimulation guideline for stimulating a vagus nerve may include information such as the current, frequency, duty ratio, stimulation time of electrical stimulation. An electrical stimulation guideline including predetermined information about electrical stimulation, as described above, is input through an input unit of an apparatus for applying electrical stimulation to a vagus nerve. As a method of inputting an electrical stimulation guideline, a user may manually input information about electrical stimulation or it may be possible to automatically receive an electrical stimulation guideline through a code (a barcode or a QR code) or an information storage device that includes information about electrical stimulation.

Next, an initial stimulation setting step is performed.

Initial stimulation values are values setting an electrical stimulation guideline as electrical stimulation in a range suitable for a user by reflecting sensitivity to electrical stimulation. Sensitivity for electrical stimulation is a concept coming from the fact that people have different senses of pain (pain) or discomfort in response to the same electrical stimulation, and means the degree to which one can easily perceive pain or discomfort in response to electrical stimulation. In the present disclosure, sensitivity to electrical stimulation reflects not only sensitivity depending on the intensity of physical stimulation and sensitivity that human perceives as pain, but also sensitivity to psychological discomfort or anxiety even though pain is not felt. When the sensitivity to electrical stimulation is high, the possibility of feeling pain or discomfort to the same electrical stimulation is high, and particularly, as a current increases, more pain or discomfort is felt.

The present disclosure applies a Stimulation Perception Index (SPI) as an index quantifying sensitivity to electrical stimulation. In the present disclosure, an SPI is calculated by applying demographic markers and/or environmental markers. Demographic markers are markers that statistically quantify sensitivity to electrical stimulation on the basis of biometric information such as age, sex, Body Mass Index (BMI), presence of medication, and presence of disease. For example, the sensitivity to electrical stimulation is higher in children and the elderly than in young adults, higher in women than in men, and lower as the BMI increases. Demographic markers are statistically organized so that this information can be applied to the SPI index. Environmental markers are markers that statistically represent sensitivity to electrical stimulation on the basis of environmental information such as temperature, humidity, illuminance, and a discomfort index. For example, when temperature or humidity is lower than a specific numerical value, sensitivity to electrical stimulation is high, when illuminance is low, sensitivity to electrical stimulation is high, and sensitivity to electrical stimulation changes, depending on the range of a discomfort index. When sensitivity to electrical stimulation indicated by an SPI index increases, this is the case when it is easy to feel pain or discomfort from electrical stimulation. Accordingly, initial stimulation values are set to decrease the degree of stimulation so that a user does not feel pain or discomfort, and for example, it may be possible to decrease the intensity of a current. However, a value that can provide a predetermined effect is provided for an electrical stimulation guideline, and when only the intensity of a current is decreased, sufficient stimulation is not applied, so the effect by application of electrical stimulation may not be obtained. Accordingly, compensation is needed as much as reducing a current and it is possible to apply a method of increasing a duty ratio in proportion to reduction of a current. The frequency of an electrical stimulation guideline may not be changed, and when the time for which electrical stimulation is applied is increased, discomfort of a user increases, so the application time of electrical stimulation may be maintained. Sensitivity to electrical stimulation is evaluated high only when an SPI is larger than a predetermined value, so it is possible to set initial stimulation values such that a current decreases and a duty ratio increases in proportion to an excess value. On the other hand, when an SPI is a predetermined value or less, it is possible to apply the current and the duty ratio of an electrical stimulation guideline as initial stimulation values. As described above, the process of deriving initial stimulation values from an input electrical stimulation guideline is performed by the initial stimulation value setter, and the initial stimulation value setter may include a DB, etc. for deriving an SPI, may include an input device for receiving age, sex, etc. to apply demographic markers, and may include an BMI measurer to directly measure and apply a BMI. Further, the initial stimulation value setter may include an input device for receiving information to apply environmental markers and may include a thermometer or a hygrometer for directly measuring and reflecting temperature, humidity, etc.

Further, an electrical stimulation starting step of applying an initial input value derived using an SPI index to an electrode is performed.

Thereafter, the present disclosure does not just maintain the initial input value as it is and performs an intra-stimulation adjustment step of adjusting electrical stimulation that is applied to an electrode.

In the present disclosure, an initial stimulation value reflects sensitivity to electrical stimulation, but it may have a bad influence on a human body when electrical stimulation is continued. Influence on a human body, as described above, may be directly recognized in the form of pain by a user, but may be revealed in a mental or emotional type such as discomfort or anxiety. When a user keeps receive electrical stimulation while bearing pain or keeps receives electrical stimulation while feeling discomfort or anxiety, positive effects such as attenuation of symptoms and relief of stress that were supposed to be obtained through electrical stimulation may not be obtained, and conversely, negative results such as worsening symptoms or increased stress may be obtained.

Accordingly, an intra-stimulation adjustment step of adjusting electrical stimulation that is applied to an electrode by applying the amount of variation of an FSWR calculated by measuring a brain wave of a user as information for checking the influence of electrical stimulation on a human body is performed in a first embodiment of the present disclosure.

The FSWR (Fast-to-Slow Wave Ratio) applied in this embodiment is a value calculated by dividing the power of a fast wave band with a relatively high frequency by the power of a slow wave band with a relatively low frequency in a brain wave measured when electrical stimulation is applied, and was developed on the purpose of checking the user's condition in response to electrical stimulation, such as when the user feels pain, discomfort, or anxiety, through the amount of variation thereof. In this case, the fast wave band may be a brain wave exceeding 13Hz and the slow wave band may be a brain wave of 13Hz or less. In detail, an FSWR change rate that is the ratio of change from an FSWR₀ derived from a brain wave measured before or immediately after electrical stimulation is applied to an FSWR_{N} derived from a brain wave measured after electrical stimulation is applied is applied to apply the amount of variation of an FSWR. When an FSWR change rate is larger than a predetermined value, it means that electrical stimulation caused a negative condition to a user even though the user cannot directly feel pain, etc., and accordingly, the intensity of a current that is applied to an electrode is decreased. In this case, when the intensity of a current is decreased, sufficient stimulation is not applied, so the effects by application of electrical stimulation may not be obtained. Accordingly, compensation is needed as much as reducing a current and it is possible to apply a method of increasing a duty ratio in proportion to reduction of a current. The frequency of an electrical stimulation guideline may not be changed, and when the time for which electrical stimulation is applied is increased, discomfort of a user increases, so the application time of electrical stimulation may be maintained. In detail, when an FSWR change rate increases 50% or more, the intra-stimulation adjustment step may be performed to decrease the current of electrical stimulation into the range of 10 ~ 30% and increase the duty ratio into the range of 10 ~45%. The intra-stimulation adjustment step is performed by an intra-stimulation adjuster and the intra-stimulation adjuster may include a brain wave measurement device that measures brain waves to derive an FSWR change rate and a storage device for storing brain wave data and deriving an FSWR and a change rate thereof, and may include a controller for controlling electrical stimulation that is applied to an electrode by reflecting an FSWR change rate.

Further, an intra-stimulation adjustment step of adjusting electrical stimulation that is applied to an electrode by applying the amount of variation of an FSBR calculated by measuring heart rate variability of a user as information for checking the influence of electrical stimulation on a human body is performed in a second embodiment of the present disclosure. Heart rate variability that means the degree of variation of heartbeat means fine variation between one heart cycle and the next heart cycle. Meanwhile, a pulse, though different at measurement positions, is the same as heartbeat or can be used instead of heartbeat in that it reflects the characteristics of heartbeat. Accordingly, pulse variability can be used instead of heart rate variability or used almost the same, and measurement of heart rate variability can be replaced with measurement of pulse variability.

The FSBR (Fast-to-Slow Beat Ratio) applied in this embodiment is a value calculated by dividing the power of a high frequency band with a relatively high frequency by the power of a low frequency band with a relatively low frequency in heart rate variability measured when electrical stimulation is applied, and was developed on the purpose of checking the user's condition in response to electrical stimulation, such as when the user feels pain, discomfort, or anxiety, through the amount of variation thereof. In this case, the high frequency band may be the range of 0.15Hz or more and 0.4Hz or less and the low frequency band is the range of 0.04Hz or more and less than 0.15Hz. In detail, an FSBR change rate that is the ratio of change from an FSBR₀ derived from heart rate variability measured before or immediately after electrical stimulation is applied to an FSWR_{N} derived from heart rate variability measured after electrical stimulation is applied is applied to apply the amount of variation of an FSBR. When an FSBR change rate is larger than a predetermined value, it means that electrical stimulation caused a negative condition to a user even though the user cannot directly feel pain, etc., and accordingly, the intensity of a current that is applied to an electrode is decreased. In this case, when the intensity of a current is decreased, sufficient stimulation is not applied, so the effects by application of electrical stimulation may not be obtained. Accordingly, compensation is needed as much as reducing a current and it is possible to apply a method of increasing a duty ratio in proportion to reduction of a current. The frequency of an electrical stimulation guideline may not be changed, and when the time for which electrical stimulation is applied is increased, discomfort of a user increases, so the application time of electrical stimulation may be maintained. In detail, when an FSBR change rate increases 50% or more, the intra-stimulation adjustment step may be performed to decrease the current of electrical stimulation into the range of 10 ~ 30% and increase the duty ratio into the range of 10 ~45%. The intra-stimulation adjustment step is performed by an intra-stimulation adjuster and the intra-stimulation adjuster may include an electrocardiogram machine that measures heart rate variability to derive an FSBR change rate and a storage device for storing electrocardiogram data and deriving an FSBR and a change rate thereof, and may include a controller for controlling electrical stimulation that is applied to an electrode by reflecting an FSBR change rate.

Exemplary embodiments of the present disclosure were described above, but it would be understood by those skilled in the art that these embodiments described above are only examples of the spirit of the present disclosure and the present disclosure may be changed in various ways without departing from the spirit of the present disclosure. Accordingly, the protective range of the present disclosure should be construed on the basis of claims rather than specific embodiments and all of spirits within the equivalent range should be construed as being included in the range of right of the present disclosure.

## Claims

1. A method for applying electrical stimulation to a vagus nerve that is a method for applying electrical stimulation to a vagus nerve through an electrode that is in contact with the skin of a human body, the method comprising:
an electrical stimulation guideline input step of inputting an electrical stimulation guideline comprising numerical values for electrical stimulation;
an initial stimulation value setting step of deriving initial stimulation values adjusting the numerical values comprised in the electrical stimulation guideline by applying an SPI calculated by applying one or more of demographic markers and environmental markers before electrical stimulation is applied;
an electrical stimulation starting step of applying electrical stimulation on the basis of the initial stimulation values; and
an intra-stimulation adjustment step of adjusting electrical stimulation that is applied to the electrode by measuring a biological signal of a user with electrical stimulation applied to the user,
wherein the SPI is an index quantifying sensitivity to electrical stimulation, the demographic markers are markers statistically reflecting sensitivity to electrical stimulation, and the environmental markers are markers reflecting environmental factors influencing sensitivity to electrical stimulation.

2. The method of claim 1, wherein the demographic markers comprise one or more pieces of information of age, sex, BMI, presence of medication, and presence of disease.

3. The method of claim 1, wherein the environmental markers comprise one or more pieces of information of temperature, humidity, illuminance, and a discomfort index.

4. The method of claim 1, wherein, in the initial stimulation value setting step, the input electrical stimulation guideline is not changed when the SPI is a predetermined value or less, and the electrical stimulation guideline is changed in proportion to an excess value of the SPI when the SPI exceeds the predetermined value.

5. The method of claim 4, wherein the electrical stimulation guideline comprises information about a current and a duty ratio, and the current of the electrical stimulation guideline is decreased and the duty ratio of the electrical stimulation guideline is increased in proportion to the excess value of the SPI in the initial stimulation value setting step.

6. The method of claim 1, wherein the biological signal of the user measured in the intra-stimulation adjustment step is a brain wave or heart rate variability.

7. The method of claim 6, wherein the intra-stimulation adjustment step adjusts electrical stimulation that is applied to the electrode by applying the amount of variation of an FSWR calculated by measuring a brain wave of a user with electrical stimulation applied to the user; and
the amount of variation of the FSWR is for checking influence on a human body by electrical stimulation applied to a user and the FSWR is calculated by dividing power of a fast wave band with a relatively high frequency by power of a slow wave band with a relatively low frequency in a measured brain wave.

8. The method of claim 6, wherein the fast wave band is a brain wave exceeding 13Hz and the slow wave band is a brain wave of 13Hz or less.

9. The method of claim 6, wherein the amount of variation of the FSWR uses an FSWR change rate that is a ratio of change from an FSWR₀ measured before or immediately after electrical stimulation is applied to an FSWR_{N} measured after electrical stimulation is applied, and
when the FSWR change rate is larger than a predetermined value, electrical stimulation that is applied through the electrode is adjusted.

10. The method of claim 9, wherein when the FSWR change rate is larger than the predetermined value, a current of electrical stimulation that is applied through the electrode is decreased and a duty ratio is increased.

11. The method of claim 10, wherein when the FSWR change rate increases over 50%, the current of electrical stimulation is decreased into a range of 10 ~ 30% and the duty ratio is increased into a range of 10 ~ 45%.

12. The method of claim 6, wherein the intra-stimulation adjustment step adjusts electrical stimulation that is applied to the electrode by applying the amount of variation of an FSBR calculated by measuring heart rate variability of a user with electrical stimulation applied to the user; and
the amount of variation of the FSBR is for checking influence on a human body by electrical stimulation applied to a user and the FSBR is calculated by dividing power of a high frequency band with a relatively high frequency by power of a low frequency band with a relatively low frequency in measured heart rate variability.

13. The method of claim 12, wherein the high frequency band is a range of 0.15Hz or more and 0.4Hz or less and the low frequency band is a range of 0.04Hz or more and less than 0.15Hz.

14. The method of claim 12, wherein the amount of variation of the FSBR uses an FSBR change rate that is a ratio of change from an FSBR₀ measured before or immediately after electrical stimulation is applied to an FSBR_{N} measured after electrical stimulation is applied, and
when the FSBR change rate is larger than a predetermined value, electrical stimulation that is applied through the electrode is adjusted.

15. The method of claim 14, wherein when the FSBR change rate is larger than the predetermined value, a current of electrical stimulation that is applied through the electrode is decreased and a duty ratio is increased.

16. The method of claim 15, wherein when the FSBR change rate increases over 50%, the current of electrical stimulation is decreased into a range of 10 ~ 30% and the duty ratio is increased into a range of 10 ~ 45%.

17. A device for applying electrical stimulation to a vagus nerve, the device comprising:
an electrode configured to apply electrical stimulation in contact with the skin of a human body;
an input unit configured to receive an electrical stimulation guideline comprising numerical information about electrical stimulation;
an initial stimulation value setter configured to derive initial stimulation values adjusting the numerical information comprised in the electrical stimulation guideline by applying an SPI calculated by applying one or more of demographic markers and environmental markers before electrical stimulation is applied; and
an intra-stimulation adjuster configured to adjust electrical stimulation that is applied to the electrode by measuring a biological signal of a user with electrical stimulation applied to the user,
wherein the SPI is an index quantifying sensitivity to electrical stimulation, the demographic markers are markers statistically reflecting sensitivity to electrical stimulation, and the environmental markers are markers reflecting environmental factors influencing sensitivity to electrical stimulation.

18. The device of claim 17, wherein the demographic markers comprise one or more pieces of information of age, sex, BMI, presence of medication, and presence of disease.

19. The device of claim 17, wherein the environmental markers comprise one or more pieces of information of temperature, humidity, illuminance, and a discomfort index.

20. The device of claim 17, wherein the initial stimulation value setter does not change the input electrical stimulation guideline when the SPI is a predetermined value or less, and changes the electrical stimulation guideline in proportion to an excess value of the SPI when the SPI exceeds the predetermined value.

21. The device of claim 20, wherein the electrical stimulation guideline comprises information about a current and a duty ratio, and
the initial stimulation value setter decreases the current of the electrical stimulation guideline and increases the duty ratio of the electrical stimulation guideline in proportion to the excess value of the SPI.

22. The device of claim 17, wherein the biological signal of the user that is measured by the intra-stimulation adjuster is a brain wave or heart rate variability.

23. The device of claim 22, wherein the intra-stimulation adjuster adjusts electrical stimulation that is applied to the electrode by applying the amount of variation of an FSWR calculated by measuring a brain wave of a user with electrical stimulation applied to the user; and
the amount of variation of the FSWR is for checking influence on a human body by electrical stimulation applied to a user and the FSWR is calculated by dividing power of a fast wave band with a relatively high frequency by power of a slow wave band with a relatively low frequency in a measured brain wave.

24. The device of claim 23, wherein the fast wave band is a brain wave exceeding 13Hz and the slow wave band is a brain wave of 13Hz or less.

25. The device of claim 23, wherein the amount of variation of the FSWR uses an FSWR change rate that is a ratio of change from an FSWR₀ measured before or immediately after electrical stimulation is applied to an FSWR_{N} measured after electrical stimulation is applied, and
when the FSWR change rate is larger than a predetermined value, electrical stimulation that is applied through the electrode is adjusted.

26. The device of claim 25, wherein when the FSWR change rate is larger than the predetermined value, a current of electrical stimulation that is applied through the electrode is decreased and a duty ratio is increased.

27. The device of claim 26, wherein when the FSWR change rate increases over 50%, the current of electrical stimulation is decreased into a range of 10 ~ 30% and the duty ratio is increased into a range of 10 ~ 45%.

28. The device of claim 22, wherein the intra-stimulation adjuster adjusts electrical stimulation that is applied to the electrode by applying the amount of variation of an FSBR calculated by measuring heart rate variability of a user with electrical stimulation applied to the user; and
the amount of variation of the FSBR is for checking influence on a human body by electrical stimulation applied to a user and the FSBR is calculated by dividing power of a high frequency band with a relatively high frequency by power of a low frequency band with a relatively low frequency in measured heart rate variability.

29. The device of claim 28, wherein the high frequency band is a range of 0.15Hz or more and 0.4Hz or less and the low frequency band is a range of 0.04Hz or more and less than 0.15Hz.

30. The device of claim 28, wherein the amount of variation of the FSBR uses an FSBR change rate that is a ratio of change from an FSBR₀ measured before or immediately after electrical stimulation is applied to an FSBR_{N} measured after electrical stimulation is applied, and
when the FSBR change rate is larger than a predetermined value, electrical stimulation that is applied through the electrode is adjusted.

31. The device of claim 30, wherein when the FSBR change rate is larger than the predetermined value, a current of electrical stimulation that is applied through the electrode is decreased and a duty ratio is increased.

32. The device of claim 31, wherein when the FSBR change rate increases over 50%, the current of electrical stimulation is decreased into a range of 10 ~ 30% and the duty ratio is increased into a range of 10 ~ 45%.
